# EUROPEAN PATENT APPLICATION

(11) **EP 2 842 557 A1**
(43) Date of publication of application: **04.03.2015**
(21) Application number: 13182338.7
(22) Date of filing: 30.08.2013
(51) Int. Cl.: A61K 31/205, A61P 27/02, A61P 27/06

(54) **Mildronate in ophthalmic disorders**

(71) Applicant: SIGMA-TAU Industrie Farmaceutiche Riunite S.p.A., 00144 Roma (IT)
(72) Inventor: Pescosolido, Nicola, 00181 Rome (IT)
(74) Representative: Tagliafico, Giulia

(57) **Abstract**

The present invention relates to mildronate for use in the prevention or treatment of ophthalmic disorders associated with an abnormal cell proliferation and/or ocular neovascularization, such as for example neovascular glaucoma, and/or proliferative vitreoretinopathy. The administration is preferably by intravitreal injection.

## Description

### FIELD OF THE INVENTION

The present invention relates to the use of mildronate, for intravitreal injection, for preventing or treating ophthalmic disorders associated with an abnormal cell proliferation and/or ocular neovascularization.

In particular the present invention relates to the use of mildronate, for intravitreal injection, for preventing or treating neovascular glaucoma, and/or proliferative vitreoretinopathy.

### BACKGROUND OF THE INVENTION

Glaucoma is a condition that causes damage to the eye and gets worse over time. It's often associated with a buildup of pressure inside the eye. Glaucoma tends to be inherited and may not show up until later in life. The increased pressure, called intraocular pressure, can damage the optic nerve which transmits images to the brain. If damage to the optic nerve from high eye pressure continues, glaucoma will cause permanent loss of vision. Without treatment glaucoma can cause total permanent blindness within a few years.

Neovascular glaucoma (NVG) is an aggressive type of glaucoma, which often results in poor visual outcomes. Clinically, the three most common conditions responsible for NVG are diabetic retinopathy, central retinal vein occlusion and carotid artery obstructive disease. Most NVG patients have severe underlying systemic and ocular pathology which causes NVG as a late presentation of their primary systemic and/or ocular disease. This makes NVG very difficult to treat. Weiss and colleagues first proposed the term "neovascular glaucoma" in 1963, when they described a severe glaucoma associated with the presence of new iris and angle vessels. These patients present with elevated intraocular pressure (IOP) and neovascularization of the iris and angle, often with hyphema and other ocular findings, such as ectropion uveae. The myriad medical conditions resulting in NVG all lead to a final common pathway of profound ocular ischemia. This ischemia induces production of vasoproliferative factors, including vascular endothelial growth factor (VEGF), which diffuse anteriorly and lead to anterior segment neovascularization of the iris (NVI) and neovascularization of the angle (NVA). Patients with NVI and NVA may have normal IOP in the early stages. If left untreated, the fibrovascular scaffolding of these vessels cross the angle and IOP becomes markedly elevated, though the angle may still be gonioscopically open. Ultimately, in the absence of definitive treatment, these fibrovascular membranes contract and produce synechial angle closure and ectropion uveae with intractable elevation of IOP and damage to the optic nerve with subsequent vision loss.

The management of NVG has two main components. The first component is reduction of the IOP by both medical and surgical means. The second component, which is arguably the most critical for effective long-term treatment outcomes, is reduction of the ischemic drive that induces formation of new blood vessels. Medical control of elevated intraocular pressure includes the topical administration of agents to reduce aqueous production and increase aqueous humor outflow to lower the IOP, such as with beta-blockers, carbonic anhydrase inhibitors, prostaglandin agonists, and alpha-adrenergic agonists. Oral administration of carbonic anhydrase inhibitors is also effective in patients who tolerate this class of medications. Finally, eyes with NVG should also be treated with topical steroids and cycloplegics, particularly if significant intraocular inflammation or hyphema is present. Medical treatment of the elevated IOP encountered in NVG is temporizing, particularly if the angles are closed by synechiae. Nevertheless, controlling the IOP until more definitive treatment can take effect is important for protecting the optic nerve from damage, decreasing associated pain, and possibly improving vision secondary to IOP-dependent corneal edema. Surgical intervention is indicated when medical therapy is inadequate to control IOP, particularly if synechial angle closure from NVA has occurred. Glaucoma surgery is helpful in the early stages to control the IOP until antiangiogenic panretinal photocoagulation can take effect and for longer term IOP control if the angle is closed secondary to NVA. The key to ultimate resolution of the underlying disease process in NVG patients is control of the ischemic drive inducing neovascularization. In these cases, medical therapy, as well as antiangiogenic therapy is initiated.

The role of antiangiogenic agents in the treatment of this disease is currently becoming established. Administration of bevacizumab induces rapid resolution of iris vessels, as demonstrated angiographically. Published case reports, small case series, and treatment outcome studies of intravitreal bevacizumab for NVG suggested that early diagnosis and treatment of NVG with intravitreal bevacizumab improved NVG outcomes (Am. J. Ophthalmol. 2006;142:1054-6). However, bevacizumab-induced regression of neovascularization is often temporary and recurrence is possible, while PRP provides a more permanent reduction of the ischemic angiogenic stimulus. The treatment of neovascular glaucoma can be fraught with complications. Eyes with this condition often present with pain, significantly decreased vision, and marked intraocular inflammation. In addition, neovascular vessels are prone to bleeding with even minor manipulation due to their friable nature. NVG represents a therapeutic challenge. However, by understanding its pathogenesis and applying those principles towards its treatment, NVG can be successfully treated when diligent clinical attention is applied.

Proliferative vitreoretinopathy (PVR) is the most common complication of a retinal detachment (RD), and occurs in approximately 8-10% of patients who develop an RD. Proliferative vitreoretinopathy, despite the long name, is simply scar tissue formation within the eye. This condition has been called by many names, including massive periretinal proliferation (MPP) and massive vitreous retraction (MVR), and was finally dubbed proliferative vitreoretinopathy (PVR) by the Retina Society Terminology Committee. "Proliferative" because cells proliferate and "Vitreoretinopathy" because the problems involve the vitreous and retina. Proliferative vitreoretinopathy can be divided into multiple categories based on the configuration of the retina and the location of the scar tissue, and this categorization is used by eye care specialists to describe to one another the severity and configuration of the retina in PVR.

Mildronate (3-(2,2,2-trimethylhydrazinium)propionate; MET-88; meldonium, quaterine, CAS Registry Number: 86426-17-7) is an anti-ischemic drug developed at the Latvian Institute of Organic Synthesis. Mildronate was designed to inhibit carnitine biosynthesis in order to prevent accumulation of cytotoxic intermediate products of fatty acid beta-oxidation in ischemic tissues and to block this highly oxygen-consuming process. Mildronate is efficient in the treatment of heart ischemia and its consequences. Extensive evaluation of pharmacological activities of mildronate revealed its beneficial effect on cerebral circulation disorders and central nervous system (CNS) functions.

The use of mildronate in the medical field is already known.

In Behav. Brain Res. 2011 Sep 12;222(1):26-32 it is described the use of Mildronate for improving functional recovery following middle cerebral artery occlusion in rats.

In Eur. J. Pharmacol. 2011 May 11;658(2-3):277-83 it is described the antidiabetic effects of mildronate alone or in combination with metformin in obese Zucker rats.

In Basic Clin. Pharmacol. Toxicol. 2009 Dec;105(6):387-9 it is described the effects of long-term mildronate treatment on cardiac and liver functions in rats.

None of these publications mention or suggest the use of mildronate for intravitreal injection for use in the treatment of neovascular glaucoma.

### SUMMARY OF THE INVENTION

The present invention relates to the use of mildronate, for intravitreal injection, for preventing or treating ophthalmic diseases or disorders associated with an abnormal cell proliferation and/or ocular neovascularization such as, for example, neovascular glaucoma, and/or proliferative vitreoretinopathy.

It is therefore an object of the present invention mildronate for use in the prevention or treatment of ophthalmic diseases or disorders associated with an abnormal cell proliferation.

It is a further object of the present invention an ophthalmic composition for intravitreal injection comprising as active ingredient mildronate and one or more diluent and/or excipient ophthalmologically acceptable, for use in the prevention or treatment of ophthalmic diseases or disorders associated with an abnormal cell proliferation.

It is a further object of the present invention an ophthalmic composition for intravitreal injection, comprising as active ingredient mildronate and one or more diluent and/or excipient ophtalmologically acceptable, for use in the prevention and/or treatment of neovascular glaucoma and/or proliferative vitreoretinopathy. It is a further object of the present invention mildronate, for intravitreal injection of a volume of 10-500 µl having a concentration in mildronate of 0.1-5.0 millimolar (in a liquid formulation) for use in the prevention or treatment of neovascular glaucoma and/or proliferative vitreoretinopathy.

It is a further object of the present invention mildronate for intravitreal injection of 100 µl of a 2.0 millimolar liquid formulation, for use in the prevention or treatment of neovascular glaucoma and/or proliferative vitreoretinopathy.

The liquid formulation according to the present invention can be any liquid formulation ophthalmologically acceptable suitable for intravitreal injection. Said liquid formulation can be, for example, a (clear) solution or a suspension in water or saline and may further contains suitable regulator of the osmotic pressure well known in the art.

The preferred route of administration of the liquid formulations and/or compositions disclosed herein is by intravitreal injection, whereby an aqueous/saline solution of mildronate is injected directly into the vitreous body located within the posterior chamber of the eye. Other method of administration of mildronate according to the present invention comprise, and are not limited to, eye drops and ointments.

The liquid formulation for intravitreal injection according to the present invention can be injected/delivered using any suitable needle, and for example a needle for insulin subcutaneous administration.

Still further aspects and elements of the present invention will become apparent to those of skill in the art upon reading and understanding of the following detailed description and examples.

### DISCUSSION OF THE DRAWINGS

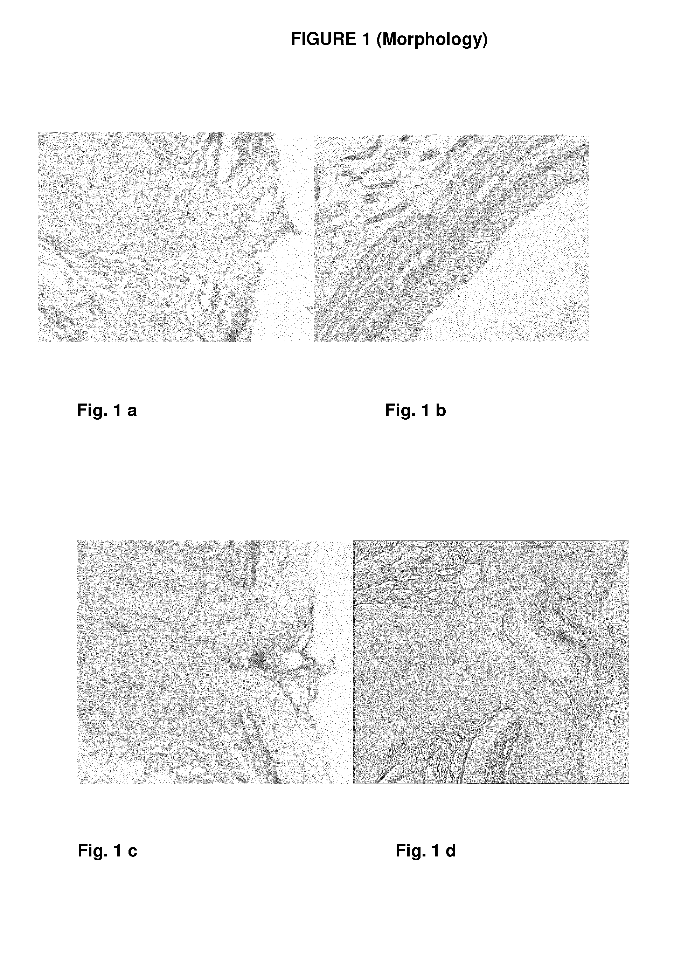

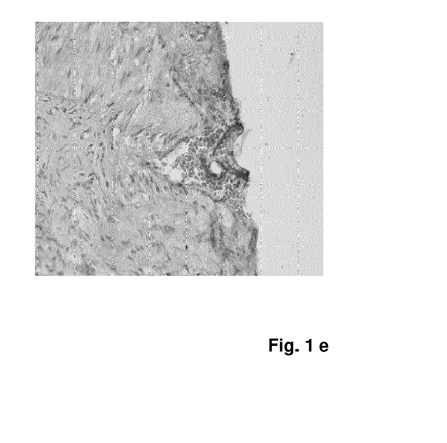

**Figure 1** **(morphology)**
   a) Section of optic nerve from control rats (50X);
   b) Section of retina from control rats (50X);
   c) Section of optic nerve from 5 mM mildronate-treated rats (50X);
   d) Section of optic nerve from 2% MTC treated rats (50X);
   e) Section of optic nerve from 2 mM mildronate + 0.6 mM L-carnitine - treated rats (50X).

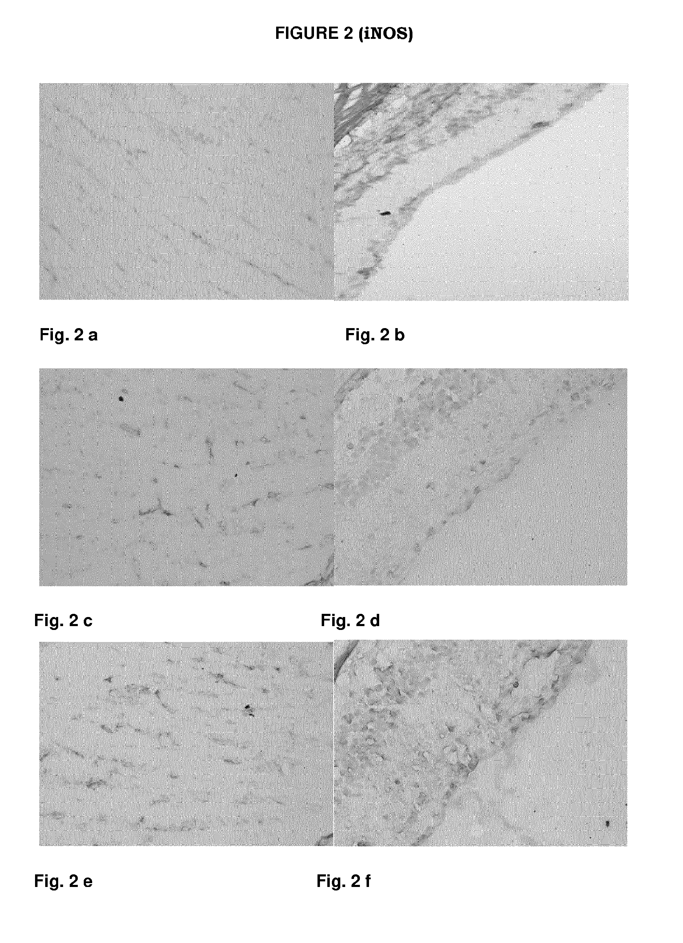

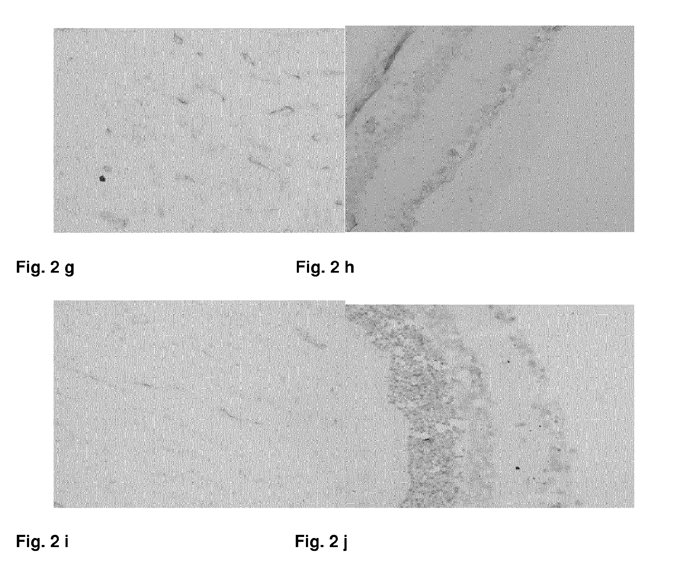

**Figure 2** **(iNOS)**
   a) Section of optic nerve from control rats (500X);
   b) Section of retina from control rats (500X);
   c) Section of optic nerve from 5 mM mildronate-treated rats (500X);
   d) Section of retina from 5 mM mildronate-treated rats (500X);
   e) Section of optic nerve from 2% MTC -treated rats (500X);
   f) Section of retina from 2% MTC -treated rats (500X);
   g) Section of optic nerve from 2 mM mildronate-treated rats (500X);
   h) Section of retina from 2 mM mildronate-treated rats (500X);
   i) Section of optic nerve from 2 mM mildronate + 0.6 mM L-carnitine-treated rats (500X);
   j) Section of retina from 2 mM mildronate + 0.6 mM L-carnitine-treated rats (500X);

### DETAILED DESCRIPTION OF THE INVENTION

The following examples further illustrate the invention without limiting it.

### EXAMPLE 1

### Mildronate for the reducing eye scar due to ocular hypertension.

Retina and optic nerve from rats treated with mildronate and/or carnitine were compared in an experimental ocular hypertension model (Acta Ophthalmol. Scand. 76 Suppl. 227: S20-1). This model has been proved to reproduce series of clinical and histopathological signs of acute human glaucoma.

### MATERIALS AND METHODS

### Animals, treatment and experimental procedures

All experimental procedures were in accordance with the ARVO Statement for the Use of Animal in Ophthalmic and Vision Research, the guidelines of the European Communities Council Directive of 24 November 1986 (86/609/EU), Italian Health Ministry guidelines, and EU directive 2010/63/EU for animal experiments.

A group of 55 male Wistar rats, average weight 300-350 g, were housed in controlled light and temperature with unlimited access to food and water. 20 animals were submitted to intraocular injection of 0.5 mM, 2 mM, 3.5 mM, 5 mM mildronate, 15 µl in physiological solution, to standardize experimental conditions (data not shown). A group of 5 animals were submitted to intraocular injection of 2% methylcellulose (MTC), 15 µl in physiological solution. MTC is a molecule capable of mechanically blocking the outflow of aqueous humor leading to ocular hypertension.

Thirty animals were randomly separated into 3 experimental groups: rats from first group received mildronate 5 mM; rats from second group received mildronate 2 mM; rats from third group received mildronate 2 mM + L-carnitine 0.6 mM, 15 µl in physiological solution; all controlateral eyes were used as control.

After local anesthesia with 0.4% oxybuprocaine eye drops (Novesina™, Novartis, Basel, Switzerland) and after induction with ether, animal were injected in the posterior chamber of the right eye with solutions described above.

The animal sufferance was evaluated by the behavioral Irwin test and by the recovery of body weight. The ocular inflammation was evaluated with Drize test adapted to the rat.

24 hours after the intraocular injection, rats were given general anesthesia by intra-peritoneal injection of 500 pl sodium-thiopental solution (Farmotal™, Pharmacia & Upjohn, USA) in saline. They were then sacrificed by carotid bleeding and eyeballs were removed.

The studies were conducted using both immunohistochemical techniques and western blot. We looked for the following on the rat retina and optic nerve head: glial activation markers, alteration of the cell cycle, apoptotic cell damage.

### Induction of the intra-ocular hypertension

To induce ocular hypertension was adapted an experimental model of chronic hypertension in vivo to a condition of acute glaucoma in rat. The animals were injected in the anterior chamber of the right eye with 15 µl of 2% (w/v) methylcellulose [(MTC) Sigma-Aldrich] in physiological solution. The contralateral eye was used as control and injected only with physiological solution. The intra-ocular pressure (IOP) was monitored at the indicated time points by tonometry (Tonopen® XL, Automated Ophthalmics, Ellicott City, MD, USA). The degree of animal sufferance was evaluated by the behavioral Irwin test and by the recovery of bodyweight. Ocular inflammation was assessed by the Drize test adapted to the rat (J. Pharm. Exp. Ther. 82: 377-90). Intra-ocular pressure was monitored on 20 different animals, for each time point from time 0 to 7 days. The animals were finally sacrificed by hemorrhagic shock. No difference was observed after comparison between non-injected and sham operated animals.

### Morphological, molecular analysis of tissue sections and of retina total protein extracts - morphology techniques and sample preparation

The eyes were enucleated, the corneas were rapidly cut vertically, and the crystalline lens and vitreous humor were removed. Then the following procedures on the residual tissues were carried out: fixation in paraformaldehyde 4% for 6 h at 4°C; rapid wash with 1X PBS pH 7.4; immersion in a 30% sucrose solution in 1X PBS pH 7.4 at 4°C overnight; embedding in Killik™ embedding medium (Bio-Optica, Milan, Italy) in order to perform the cryostat cut into 10 µm sections, horizontally, to allow longitudinal observation of the optic nerve. The sections were placed on microscope slides and stored at -20°C.

### Immunohistochemistry

iNOS expression was detected with a rabbit polyclonal anti-iNOS antibody (Santa Cruz) (1:50), followed by a green fluorescent goat anti-rabbit IgG Alexa Fluor® (Molecular Probes) secondary antibody.

Caspase-3 expression was detected with a rabbit polyclonal anti-caspase-3 antibody (Santa Cruz) (1:100), followed by a green fluorescent goat anti-rabbit IgG Alexa Fluor® (Molecular Probes) secondary antibody.

The sections, left to dry at room temperature for 30 min, were subjected to various treatments: washes in 1X PBS for 5 min; post-fixation with alcohol at increasing volumes (70°, 95°, 100°) for 2 min; washes in 1X PBS for 5 min; block with Normal Rabbit Serum (NRS) or serum Normal Goat Serum (NGS), 10% in PBS, 1h in a moist chamber; incubation with the primary antibody diluted in 1X PBS + BSA 1% for 1 h; fast washes in 1X PBS; incubation with the secondary antibody diluted 1:200 1X PBS + 1% of the serum of the animal in which the antibody was produced, for 30 min at room temperature; washes in 1X PBS for 5 min; mounting of the slides with Eukitt™.

The observations were made under a fluorescence microscope and pictures taken by computerized image acquisition.

### Preparation of protein extracts

Rat retinas were immersed in lysis buffer, and test-tubes were kept for 10 minutes on ice, then centrifuged at 13000 rpm for 5 sec and transferred back to the ice for another 10 min. Finally, they were centrifuged for 10 min, at 13000 rpm. Subsequently, the supernatant was collected and used in the study.

### Western blotting and immunodetection

An equal amount of protein was loaded, separated of a SDS PAGE gel and then iNOS and caspase-3 presence was identified by Western blotting. α-tubulin was used as loading control to normalize protein expression. The Bradford® assay was used to estimate protein concentration.

### Transferase (TdT)-mediated dUTP nick end labeling (TUNEL) assay

The sections (thickness of 10 µm), left to dry at room temperature for 30 min, were subjected to the following treatments: rehydration through a descending alcohol series (100°, 95°, 75°; 2 min each); incubation with protease 20 µg/mL in Triton X-100 to 0.1% at 37°C for 30 min; washes with 1X PBS for 2 min; incubation with TUNEL reagent (In Situ Cell Death Detection Kit, AP/ROCHE), containing 1/10 in volume of terminal deoxynucleotidyl transferase and the remaining fraction of nucleotides labeled with a fluorescent chromophore, 10 µl per section at 37°C for 1h. On each slide, one of the sections was always used as the negative control. The observations were made under a fluorescence microscope and pictures were taken by computerized image acquisition.

### Statistical analysis

All the experiments were repeated at least four times. Statistical analysis of the results was performed using the Analysis Of Variance (ANOVA) test. The significance of the results was determined by the Student's t-test. P-values <0.05 were considered significant.

### RESULTS AND DISCUSSION

### Cellular morphology

In the controls optic nerve fibers are tightly arranged (Fig. 1 a) and surrounded by quiescent astrocytes (small and round nuclei), disposed in rows; retina also shows its characteristic morphology (Fig. 1 b). By contrast, optic nerves from animals treated with mildronate 5 mM fibers do not run longitudinally, the papilla shows an excavation or "cupping" and the majority of astrocytes shows drumstick-shaped and expanded nuclei; both nuclear hypertrophy and chromatin fragmentation are considered as cell activation sign in the reduction of the "production" of scar in the eye (this is a clear indication that mildronate is useful for preventing or treating ophthalmic diseases or disorders associated with an abnormal cell proliferation such as neovascular glaucoma and/or proliferative vitreoretinopathy).

In retinas from mildronate treated animals, moreover, picnotic and/or vacuolated nuclei and fragmented chromatin are found in glial cells of the nerve fiber layer, in Muller cells and in neurons of ganglionic cell layer; these alterations suggest that retinal cells and astrocytes are undergoing to cell death , thus reducing the onset/formation of scar in the eye (therefore this is a clear indication that mildronate is useful for preventing or treating ophthalmic diseases or disorders associated with an abnormal cell proliferation, such as neovascular glaucoma and/or proliferative vitreoretinopathy).

These data are similar to results obtained from 2% methylcellulose (MTC) injection in the anterior chamber (induction of ocular hypertonicity) (Fig. 1 d). Tissues treated with mildronate and L-carnitine maintained a more regular organization of astrocytes and the papilla is similar to the control (Fig. 1 e).

### DNA damage

Eyes from control animals showed no positivity both in optic nerve and retina.

The sections of eyes from mildronate 5 mM-treated animals showed a strong fluorescence in the RGCs and astrocytes cells of the optic nerve due to DNA fragmentation.

The sections of eyes from mildronate 2 mM-treated animals showed a slight positive fluorescence and specimens from animals treated both with mildronate and carnitine were quite similar to control, suggesting a cytoprotective effect for carnitine (antagonistic effect vs. mildronate).

### iNOS expression

Nitric oxide synthases (NOSs) are a family of enzymes catalyzing the production of nitric oxide (NO) from L-arginine. The inducible isoform, iNOS, is involved in immune response, binds calmodulin at physiologically relevant concentrations, and produces NO as an immune defense mechanism, as NO is a free radical with an unpaired electron. iNOS immunolocalization was performed in order to verify cellular stress in optic nerve and retina at different concentrations of mildronate.

Literature data show that iNOS activation and high levels of NO are produced after an ischemic insult. High NO concentrations are cytotoxic and cause lipid peroxidation. It was also shown that iNOS expression is responsible for the death of retinal neuronal cells in vitro, while in vivo has been proved that it induces apoptosis in the inner nuclear layer of the retina.

24 hours after treatment with 5 mM mildronate high levels of iNOS were expressed both in glial retinal cells (Fig. 2 d) and astrocytes of optic nerve (Fig. 2 c) as compared to control (Fig. 2 a, Fig 2 b), suggesting that treatment with mildronate leads to high induction of iNOS.

In specimens from MTC -treated rats, there were high levels of iNOS both in glial retinal cells (Fig. 2 d) and astrocytes of optic nerve (Fig. 2 c), as well as for 5 mM mildronate-treated specimens.

Both in optic nerve (Fig. 2 g) and the retina (Fig. 2 h) sections from 2mM mildronate-treated rats showed a mild decrease of iNOS expression with respect to 5 mM mildronate-treated specimens.

Mildronate + L-carnitine treatment decreases iNOS expression (Fig. 2 i, Fig. 2 j).

Data were confirmed by western blot analysis of total protein extracts of retina.

### Caspase-3 expression

Caspase-3 immuodetection was performed to verify if apoptosis plays a role in cell death observed by TUNEL assay.

Specimens from both 5 mM and 2 mM mildronate, MTC treated, and 2 mM mildronate + L-carnitine- treated were tested to investigate if L-carnitine administered in combination with mildronate could enhance ganglionic cells and astrocytes survival by modulating caspase-3 activity.

Both in astrocytes and retinal cells from 5 mM mildronate treated eyes as well as MTC treated eyes, the high caspase-3 levels suggest an apoptotic program activation via mitochondrial pathway.

2 mM mildronate treated eyes showed a mild decrease of positivity both in optic nerve and retina.

In specimens treated with 2 mM mildronate + L-carnitine the staining pattern was quite similar to that found in control. This was probably due to the carnitine protection against cell damage through the competition with the mildronate (e.g. antagonistic action of L-carnitine vs. the therapeutic activity of mildronate). Data were confirmed by western blot analysis of total protein extracts of retina.

The results above reported above show that:
- both retinal ganglion cells and optic nerve astrocytes were very sensitive to mildronate and showed apoptosis 24 hours after treatment;
- treatment with mildronate increased NO levels and iNOS immunolocalization.

It is evident that these unexpected results indicate that mildronate is useful for preventing or treating ophthalmic diseases or disorders associated with an abnormal cell proliferation and/or ocular neovascularization, such as neovascular glaucoma and/or proliferative vitreoretinopathy.

Moreover, the results reported above also show that the simultaneous administration of L-carnitine inhibited the pharmacological activity of mildronate. The results reported above were unexpected in view of the existing prior art.

The preferred injectable aqueous/saline liquid formulation of mildronate may contain, certain inactive ingredients which cause the solution to be substantially hypotonic, isotonic, or hypertonic and of a pH range of 4.5-9.0 which is non-toxic for injection into the eye. Such solution for injection may be in glass vials or pre-filled syringes maintained at room temperature or refrigerated temperature, ready for use. In addition such solutions of mildronate may be initially lyophilized to a dry state and thereafter, may be reconstituted prior to use.

Mildronate (generally commercialized as inner salt) employed in the present invention can be obtained with pharmaceutical/ophthalmic purity from several manufacturers of fine chemicals.

## Claims

1. Mildronate for use in the prevention or treatment of ophthalmic diseases or disorders associated with an abnormal cell proliferation and/or ocular neovascularization.

2. Mildronate for intravitreal injection for use in the prevention or treatment of neovascular glaucoma.

3. Mildronate for intravitreal injection for use in the prevention or treatment of proliferative vitreoretinopathy.

4. The use of claims 2-3, for intravitreal injection of 10-500 µl of a 0.1-5.0 mM liquid formulation.

5. The use of claims 2-4, for intravitreal injection of 100 µl of a 2.0 mM liquid formulation.

6. The use of claims 2-5 in which the liquid formulation is in saline or distilled water.

7. The use of claims 2-6 further comprising regulator of the osmotic pressure.

8. Ophthalmic composition for intravitreal injection, comprising as active ingredient mildronate, for use in the prevention and/or treatment of neovascular glaucoma.

9. Ophthalmic composition for intravitreal injection, comprising as active ingredient mildronate, for use in the prevention and/or treatment of proliferative vitreoretinopathy.

10. The composition of claims 8-9, further comprising one or more diluent and/or excipient ophthalmologically acceptable.
